# EUROPÄISCHE PATENTSCHRIFT

(11) **EP 0 890 367 B1**
(45) Veröffentlichungstag und Bekanntmachung des Hinweises auf die Patenterteilung: **12.12.2001**
(21) Anmeldenummer: 98111535.5
(22) Anmeldetag: 22.06.1998
(51) Int. Cl.: A61L 31/00

(54) **Urologisches Implantat, insbesondere Gefässwandstütze für den Urinaltrakt**
Urologic implant, especially support for the vascular walls of the urinal tract
Implant urologique notamment support pour la paroi des vaisseaux de la sonde urinaire

(30) Priorität: 09.07.1997 DE 19729279
(43) Veröffentlichungstag der Anmeldung: 13.01.1999
(73) Patentinhaber: Hildebrandt, Peter, 90482 Nürnberg (DE)
(72) Erfinder: Hildebrandt, Peter, 90482 Nürnberg (DE)
(74) Vertreter: Hübner, Gerd, Dipl.-Phys.

(56) Entgegenhaltungen:
- DE-A- 19 533 682
- US-A- 4 527 293
- US-A- 5 180 715
- US-A- 5 328 954
- - Broschüre "Tectus - Der inkrustierungsresistente Prostatastent", UroNova GmbH, D-91058 Erlangen (DE), 4 Seiten
- - Broschüre "Memotherm - Prostatischer Stent", C.R. Bard GmbH, D-76227 Karlsruhe (DE), 2 Seiten
- - Broschüre "NIR Primo", SCIMED Life Systems Inc. (US), 1 Seite
- - Broschüre "ACS RX MULTI-LINK HP", Guidant, 1 Seite
- - Broschüre "PROSTACOIL Temporary Intraprostatic Stent", InStent (US), 2 Seiten
- - Broschüre "Ureter-Dauerschienen HEPARIUS", UROMED Kurt Drews GmbH, D-22113 Oststeinbek (DE), 4 Seiten

## Beschreibung

Die Erfindung betrifft ein urologisches Implantat, insbesondere eine Gefäßwandstütze für den Urinaltrakt, wie z.B. einen Urethral-, Prostata- oder Harnleiter-Stent, mit einem im wesentlichen röhrenförmigen, eigenstabilen Träger. Solche Gefäßwandstützen, die im Fachjargon üblicherweise als "Stents" bezeichnet werden, werden in der Regel zur Aufweitung und Offenhaltung von krankhaft verschlossenen oder im Durchmesser verengten Körpergefäßen verwendet. Als weitere Anwendungsbeispiele für die angegebenen urologischen Implantate sind Urinal-Katheter, künstliche Harnleiter, entsprechend durch eine Blase ergänzte künstliche Harnableitesysteme usw. zu nennen.

Im folgenden soll der Einfachheit halber bei der Erörterung des Standes der Technik und der Erfindung auf die bereits erwähnten Stents beispielhaft eingegangen werden:

Urethral-, Prostata- und Harnleiter-Stents sind für Patienten mit Urinal-trakt-Störungen unverzichtbare Implantate, falls die Patienten nicht in der Verfassung für einen entsprechenden operativen Eingriff sind. Eine große Auswahl an Stent-Ausgestaltungen und Materialien sind heute bereits verfügbar. Ausgestaltungen und Material richten sich in der Regel nach dem Einsatzort des Stents und der verwendeten Implantationstechnik. So sind beispielsweise röhrenförmige Träger, die aus einem expandierbaren Gittermaterial gefertigt sind, üblich. Der Stent wird im kontrahierten Zustand über ein Katheter in das entsprechende Gefäß eingeführt, dort beispielsweise durch Einwirkung einer Blase unter Ausdehnung seiner Gitterstruktur expandiert und weitet das Gefäß damit auf. Es verbleibt am Implantationsort und dient zur Abstützung der Gefäßwand gegen eine abermalige Verengung.

Als schwerwiegendes Problem treten bei allen bisher eingesetzten und getesteten Materialien für Urinaltrakt-Stents bereits bei mehrwöchigem Kontakt mit Urin Kristallisationsprozesse und Bakterienbelag an der StentOberfläche auf. Dies zu schwerwiegenden Funktionsstörungen der Implantate, was den medizinischen Erfolg der Stent-Implantation nachhaltig gefährdet. Zahlreiche Untersuchungen haben sich in Hinsicht auf dieses Problem mit verschiedenen medizintechnischen Werkstoffen, wie Titan, Edelstählen und Polymeren befaßt. Es wurde jedoch bei noch keinem Material eine wirklich dauerhafte Kristallisationsresistenz nachgewiesen.

Um dieses Problem zu lösen, zeigt der Stand der Technik verschiedene Ansätze. So offenbart die US-A-4 527 293 ein urologisches Implantat, dessen Oberfläche mit einer Hydrogel-Beschichtung versehen ist. Dieses Hydrogel-Material zeigt ein Schrumpf/Quell-Verhalten, das bei einer wechselnden Urin-Zusammensetzung so variiert, daß das Hydrogel-Polymer mit hoher Ansprechgeschwindigkeit auf eine solche Veränderung einem Quellen und Kollabieren unterzogen wird. Dadurch werden Inkrstieungen auf dem Implantat verhindert.

Als technologischer Hintergrund ist im übrigen auf die US-A-5 180 715 hinzuweisen, die ein Verfahren zur Behandlung von Urinaltrakt-Infektionen u.dgl. betrifft. Dabei wird grundsätzlich die Spülung des infizierten Organs, wie beispielsweise der Harnblase, mit Glycosaminoglycanen vorschlägt, um ein Anhaften von Bakterien an den Schleimhautzellen des Organs zu verhindern.

Ein weiteres Beispiel für entsprechende Maßnahmen aus dem Stand der Technik zur Erhöhung der Kristallisations-resistenz ist dem US-Patent 5,328,954 entnehmbar. In dieser Druckschrift ist eine Inkrustierungs- und bakteriell-resistente Beschichtung zur Anwendung bei medizinischen Vorrichtungen offenbart. Die Beschichtung umfaßt ein Reaktionsprodukt, das aus einer kovalenten Bindung eines hydrophilen Polyurethan-Präpolymers und Aminopolycarboxyl-Säure gebildet ist. Ein Urease-Inhibitor und/oder ein antibakterieller Wirkstoffkönnen der Beschichtung ebenfalls zugeführt werden.

Das Ziel dieser Beschichtung - nämlich Oberflächen resistent gegenüber Inkrustierungen zu machen - wird dort über antibakterielle Stoffe verfolgt, die an der Oberfläche gebunden werden und dort eine pH-Wert-Erhöhung, wie sie durch Bakterienbelag verursacht wird, verhindern sollen. Inkrustierungen sollen dabei dadurch blockiert werden, daß eine Fällung der Urinkomponenten aufgrund eines erhöhten pH-Wertes nicht mehr stattfindet.

Bei den Stoffen, die in der Druckschrift für die Immobilisierung angegeben werden, handelt es sich unter anderem um Ethylendiamintetraacetat-Komplexe, Antibiotika und Silberionen, die ionisch oder kovalent an Polyurethane gebunden werden. Wie sich jedoch gezeigt hat, ist die mit diesem Stand der Technik erreichbare Eindämmung von Inkrustierungen nach wie vor verbesserungsbedürftig. Auf die Lösung dieser Problematik zielt die vorliegende Erfindung ab.

Der Erfindung liegen dabei natürliche biomedizinische Vorgänge zur Unterbindung des Kristallwachstums im Urinaltrakt als Ansatz zugrunde:

Die meisten ionischen Komponenten des menschlichen Urins liegen in übersättigten Konzentrationen vor. Dennoch kommt es im gesunden Urin nicht zur Fällung oder zur Bildung größerer Kristallite. Dies wird durch die Anwesenheit nieder- oder hochmolekularer Inhibitoren verhindert. Zu den niedermolekularen Inhibitoren, deren Wirksamkeit eher gering einzuschätzen ist, gehören u.a. Natrium-, Kalium-, Magnesium- und Zitrationen. Die entscheidende Rolle als Inhibitoren spielen die gegenüber dem Urin enthaltenen Makromoleküle, die sich in zwei Gruppen einteilen lassen. Es handelt sich dabei um die Glycosaminoglycane (GAG) und um Proteine. Zur ersten Gruppe zählen Heparin, Heparansulfat und verschiedene Chondroitinsulfate, zur zweiten Nephrocalcin, Osteopontin und Tamm-Horsfall-Protein. Diese genannten Makromoleküle wirken durch Adsorption an Moleküle und Kristalloberflächen und verhindern so das Anwachsen größerer Kristallstrukturen. Dabei ist die Wirkung eines Inhibitors meist auf eine oder wenige Teilchensorten beschränkt. So bindet z.B. Nephrocalcin an Kalzium-Ionen und Heparin an Oxalat-Ionen sowie -Kristalliten als Inhibenten.

Liegen nun Inhibitor und Inhibent in im Urin gelöster Form vor, so ist der Wirkungsmechanismus aktiv. Adsorbieren in Betracht kommende Urinkomponenten jedoch an Oberflächen, so können die Inhibitoren nicht wirksam werden. Aufgrund des geometrischen Aufbaus der Makromoleküle wird die Bindung zwischen Inhibitor und einem adsorbierten Inhibenten unmöglich. Die an der Oberfläche adsorbierenden Inhibenten können dann praktisch als Kristallisationskeime wirken, von denen ein Kristallisationsprozeß ausgeht. Auch mit den angelagerten Inhibenten können die Inhibitoren keine Bindung eingehen, wodurch der Kristallisationsprozeß unaufhaltsam fortschreitet.

Die Verhinderung einer chemischen Bindung in Folge der räumlichen Anordnung von Molekülen wird als sterische Hinderung bezeichnet. Da diese sterische Hinderung bei allen Oberflächen auftritt, ist damit das Kristallwachstum weitgehend unabhängig von Werkstoff- und Oberflächeneigenschaften. Eine geeignete Bindung von Inhibitoren an das Substrat erzeugt dagegen eine Schicht, an der Urinkomponenten wie in dem als Elektrolyt-Lösung zu bezeichnenden Urin gebunden werden. An solchen gebundenen Urinkomponenten können sich keine weiteren Komponenten anlagern, so daß ein Kristallwachstum blockiert wird.

Die Erfindung schlägt nun vor dem Hintergrund dieser biomedizinischen Grundlagen zur Lösung der eingangs geschilderten Problematik ein urologisches Implantat, insbesondere eine Gefäßwandstütze für den Urinaltrakt vor, bei dem laut Patentanspruch 1 an der Oberfläche des vorzugsweise im wesentlichen röhrenförmigen, eigenstabilen Trägers eine Spacer-Moleküllage und eine daran gebundene Inhibitor-Lage aus einem Glycosaminoglycan gebunden ist.

Wie im Hinblick auf die Erfindung festgestellt wurde, reicht eine direkte Bindung von Inhibitoren an der Trägeroberfläche nicht aus. Die direkte Bindung führt nämlich zu Verformungen der makromolekularen Inhibitoren, die eine Bindung zwischen Inhibitor und Inhibenten, also den kristallbildenden Urinkomponenten, verhindern. Das Einfügen des beanspruchten Spacers zwischen der Trägeroberfläche und der Inhibitor-Lage vergrößert den Abstand zwischen Trägeroberfläche und Inhibitor und kann damit die oben erläuterte Verformung der Moleküle und sterische Hinderung vermeiden.

Gemäß bevorzugten Ausführungsformen kann der Träger einerseits aus einem Polymer, wie Silikon, Polyurethan, Polyvinylchlorid oder dergleichen oder andererseits aus Tantal, eine Titanlegierung, medizinischem Stahl oder pyrolytischem Kohlenstoff, jeweils mit einer amorphen Siliciumkarbid-Beschichtung als aktiver Substratoberfläche bestehen. Auch eine Ausbildung aus einem Metallgerüst mit einer Polymer-Beschichtung aus den obengenannten Polymermaterialien ist möglich. Je nach Materialbeschaffenheit des Trägers und seiner Substratoberfläche wird auch die Auswahl für die chemischen Komponenten der Spacer-Lage getroffen. Bei einem Silikonsubstrat kann die Spacer-Lage auf der Basis einer Propylsiloxyl-Verbindung, insbesondere aus einer partiell substituierten 3-(Adipinsäure-Amido-)Propylsiloxyl-Verbindung gebildet sein.

Bei Verwendung von Trägern mit einer amorphen Siliciumkarbid-Beschichtung als aktivierter Substratoberfläche hat sich eine Spacer-Lage auf der Basis einer photoaktiven Benzophenon-Verbindung, insbesondere eine Fmoc-p-Bz-Phe-OH-Lösung in N-N'-Dimethylformamid als geeignet herausgestellt.

Bevorzugterweise wird als Inhibitor-Lage eine auf der Spacer-Lage immobilisierte Heparin-Lage verwendet. Die Heparin-Lage wird dabei beispielsweise mittels einer kovalenten Peptid-Bindung auf der Substratoberfläche angelagert und immobilisiert. Dieser Bindungstyp tritt insbesondere bei der erwähnten Realisierung der Spacer-Lage auf der Basis einer photoaktiven Benzophenon-Verbindung auf.

Zusammenfassend haben experimentelle Untersuchungen mit erfindungsgemäß ausgerüsteten Gefäßwandstützen, bei denen ein Siliciumkarbid-Spacer-Heparin-Komplex auf einem Tantal-Träger eingesetzt wird, eine starke Inhibierungswirkung und damit Unterdrückung des Kristallwachstums ergeben. So konnten bei in-vitro-Versuchen durch Eintauchen entsprechender Proben in Kunsturin über einen Zeitraum von 12 Tagen keine erkennbaren Kristallablagerungen nachgewiesen werden. Das Ergebnis solcher in-vitro-Versuche erlaubt Rückschlüsse auf das Verhalten der erfindungsgemäßen Urinaltrakt-Stents in vivo. Zeiträume von wenigen Wochen entsprechen aufgrund des in-vitro höheren Kristallisations-Potentials Zeiträumen bei in-vivo-Versuchen von mehreren Monaten.

Durch die experimentellen Untersuchungen konnte auch die Unabhängigkeit der Kristallisations-unterbindenden Wirkung der Beschichtung vom pH-Wert des Urins und eine ausreichende Langzeitstabilität der Heparin-Bindung an der Spacer-Lage nachgewiesen werden.

Weitere Merkmale, Einzelheiten und Vorteile des Erfindungsgegenstandes sind der nachfolgenden Beschreibung entnehmbar, in der Ausführungsbeispiele des Erfindungsgegenstandes anhand der beigefügten Zeichnungen näher erläutert werden. Es zeigen:
- Fig. 1 und 2: Modellzeichnungen der Bindung zwischen Inhibitor und Träger ohne und mit Spacer-Lage,
- Fig. 3: eine Ansicht einer Stent-Struktur in auf eine Ebene projizierter Darstellung und
- Fig. 4: eine schematische Darstellung der Beschichtung des Stents auf der Basis eines Siliciumkarbid-Spacer-Heparinkomplexes.

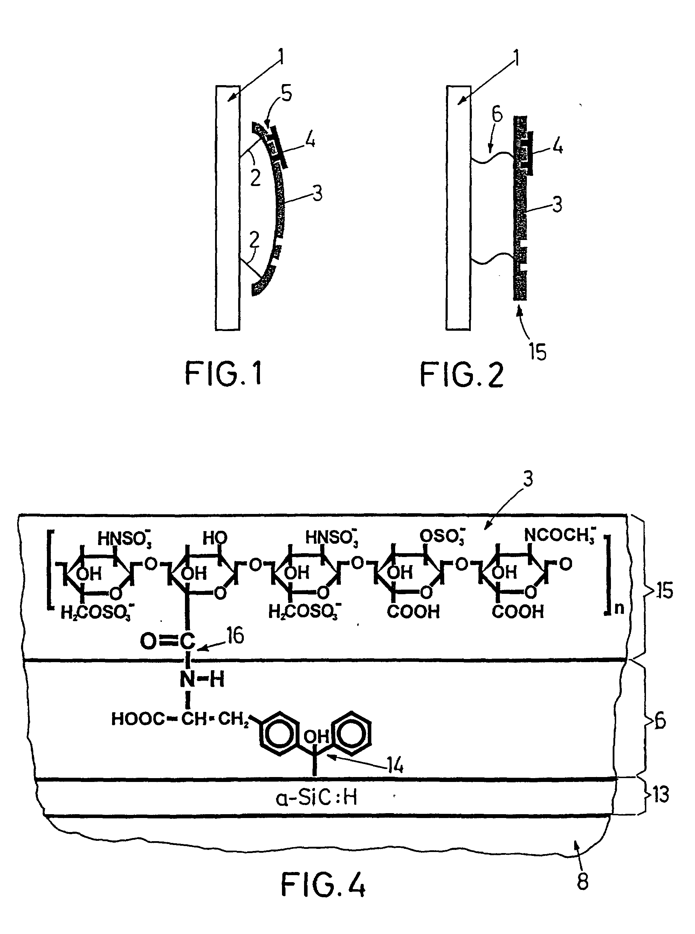

In Fig. 1 ist ein Substrat 1 dargestellt, an das über eine direkte Bindung 2 ein Inhibitor-Molekül 3 beispielsweise in Form von Heparin angelagert ist. Wie modellhaft angedeutet ist, führt die direkte Bindung 2 zu einer Verformung des Inhibitor-Moleküls 3, die wiederum dazu führt, daß sich die Urin-Komponente 4 in Form von z.B. eines Oxalat-Ions zwar an die entsprechenden Bindungsstellen des Heparins anlagern kann. Dies erfolgt jedoch in einer praktisch unvollständigen Weise, so daß die Urin-Komponente 4 ihrerseits Bindungsstellen 5 zur Verfügung stellt, an denen sich weitere Urin-Komponenten 4 anlagern können. Dies würde zu einer Kristallbildung führen.

Wie Fig. 2 modellhaft darlegen soll, wird durch die Einfügung einer Spacer-Lage 6 eine Bindung des Inhibitor-Moleküls 3 an das Substrat 1 erreicht, ohne daß eine Verformung des Inhibitor-Moleküls 3 stattfindet. Insofern kann die Urin-Komponente 4 komplett an das Inhibitor-Molekül 3 binden, ohne daß die Urin-Komponente 4 noch Bindungsstellen 5 zur Verfügung stellen könnte. Es kann also keine Anlagerung weiterer Urin-Komponenten erfolgen, so daß die Substratoberfläche durch eine derartige Beschichtung inaktiviert und resistent gegen Inkrustierungen ist.

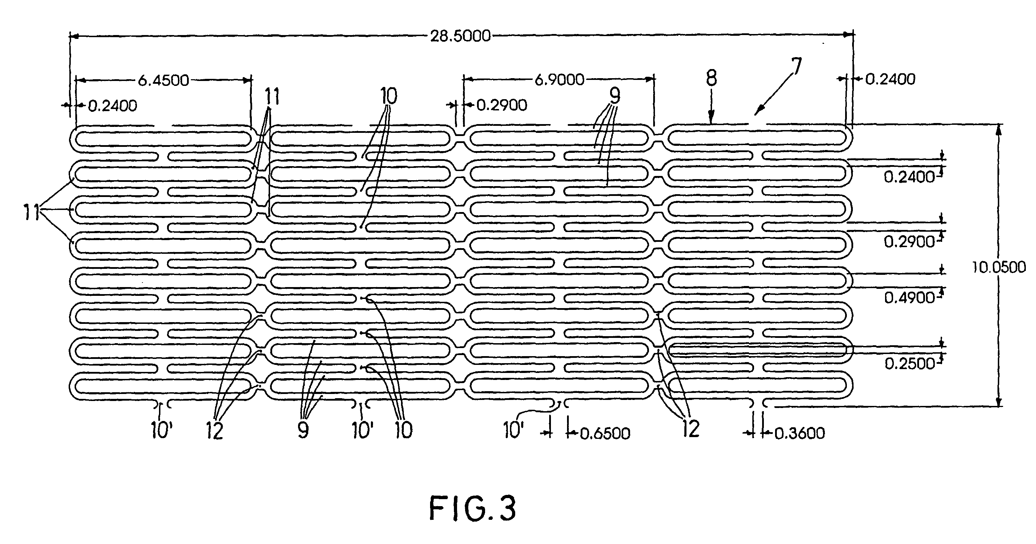

Fig. 3 zeigt nun die typische Ausgestaltung eines Harnleiter-Stents 7, der einen Träger 8 mit einer Gitterstruktur bestehend aus Längsstegen 9 und Querstegen 10 aufweist. Die Längsstege 9 sind über halbkreisförmige Endstücke 11 zu langlochförmigen Rahmeneinheiten verbunden, die wiederum über kurze Verbindungsstege 12 und die Querstege aneinander hängen. Die in Fig. 3 gewählte Darstellung zeigt quasi eine im Bereich der Querstege 10' aufgeschnittene und in die Darstellungsebene von Fig. 3 ausgerollte Gitterstruktur. Zur Deutlichmachung der Dimensionen der erfindungsgemäßen Gefäßwandstütze sind im übrigen in Fig. 3 entsprechende Abmessungen des Stents und seiner Stege in Millimeter eingetragen. Ferner ist darauf hinzuweisen, daß die in Fig. 3 gezeigte Gitterstruktur dem nicht dilatierten bzw. expandierten Zustand entspricht. Bei der Ausdehnung des Stents weiten sich die langlochförmigen Rahmen rautenförmig auf und verkürzen sich gleichzeitig.

In Fig. 4 ist der Aufbau des Silicium-Carbid-Spacer-Heparin-Komplexes auf dem Tantal-Träger 8 dargestellt. Letzterer ist mit einer Substratbeschichtung 13 aus aktiviertem Siliciumkarbid (aSiC:H) versehen. An diese Substratbeschichtung 13 ist die Spacer-Lage 6 angelagert, die in noch näher zu erörternder Weise auf der Basis einer photoaktiven Benzophenon-Bindung aufgebaut und kovalent über eine C-C- oder Si-C-Bindung 14 an die Oberfläche der Substratbeschichtung 13 gebunden ist.

Die Inhibitor-Lage 15 besteht aus Heparin-Molekülen, die über eine Peptid-Bindung 16 ihrer Carboxyl-Gruppen (O = C-O-H) an der Spacer-Lage 6 immobilisiert sind. Die Inhibitor-Lage 15 ist also durch eine flächendekkende Heparinschicht repräsentiert, die in der Lage ist, Oxalationen stabil zu binden und so eine inerte Bedeckung für die Stentoberfläche zu schaffen. Weitere Kristallisationsprozesse werden so unterbunden.

Nachfolgend wird in Form einer Auflistung einzelner Verfahrensschritte ein Beschichtungsverfahren zur Immobilisierung von Heparin auf einer aktivierten Siliciumkarbid-Beschichtung wiedergegeben. Dieses Beschichtungsverfahren ist im übrigen aus dem Stand der Technik grundsätzlich bekannt und im Ausführungsbeispiel der DE 195 33 682 A1 ausführlich beschrieben. Es bedarf also keiner nochmaligen zusammenfassenden Darlegung.

| **Beschichtungsverfahren zur Immobilisierung von Heparin auf a-SiC** | |
|---|---|
| **Probenvorbereitung** | |
| Ätzen mit 40% HF | 2 min |
| Spülen mit destilliertem Wasser | |

| **Spaceranbindung** | |
|---|---|
| Inkubieren der Probe in 2 ml Spacerlösung | |
| 10 mg Fmoc-p-Bz-Phe-OH in 2 ml Dimethylformamid (DMF) | |
| Allseitiges Belichten mit UV-Licht (365nm) | 1 min |
| Spülen mit destilliertem Wasser | |

| **Abspalten der Fmoc-Schutzgruppe** | |
|---|---|
| Inkubieren der Probe in 25% Piperidin-Lösung in DMF | 5 min unter Schütteln |
| Inkubieren der Probe in 25% Piperidin-Lösung | 15 min unter Schütteln |
| Spülen mit destilliertem Wasser | |

| **Heparinanbindung** | |
|---|---|
| Inkubieren der spacerbeschichteten Probe in 2 ml | 4 h |
| Reaktionslösung 100 mg Heparin, 10 mg Carbodiimid, 2ml destilliertes Wasser | |
| Spülen mit destilliertem Wasser | |

Als weiteres Ausführungsbeispiel für die Anlagerung einer aktiven Heparin Beschichtung auf einer Gefäßwandstütze für den Urinaltrakt wird im folgenden noch kurz das Beschichtungsverfahren zur Immobilisierung von Heparin auf Silikon dargestellt:

| **Herstellung partiell substituierter 3-Aminopropylsiloxyl-Silikon-Oberflächen** | |
|---|---|
| Probe in 50 ml Ethanol/Wasser rühren | 30 min |
| bei 40°C in 1 ml 3-Aminopropyltiethoxysilan + 49 ml Ethanol/Wasser rühren | 16 h |
| in 50 ml Wasser, | 30 min |
| in Ethanol/Wasser | 2 h |
| und wieder in Wasser rühren | 30 min |

| **Herstellung partiell substituierter 3-(Adipinsäureamido)-propylsiloxyl-Silikon-Oberflächen** | |
|---|---|
| bei 4°C in 50 ml Wasser + 365 mg Adipinsäure + 50 mg CME- | 16h |
| CDI rühren (CME-CDI:N-Cyclohexyl-N'-(2-morpholinoethyl)-carbodiimidmethyl-p-toluolsulfat) in Wasser rühren, an Luft trocknen | 30 min |

| **Heparinanbindung** | |
|---|---|
| bei 4°C in 50 ml Puffer pH 5,0 + 50 mg CME-CDI rühren | 30 min |
| in Eiswasser waschen | 30 sec |
| bei 4°C in 50 ml Puffer pH 5,0 + 100 mg Heparin rühren in Wasser waschen, an Luft trocknen lassen | 16 h |

Zu den beiden vorstehend tabellarisch wiedergegebenen Beschichtungsverfahren ist darauf hinzuweisen, daß sich diese auf Proben für ein Stent-Trägermaterial in Form einer Tantalprobe mit einer Beschichtung aus amorphem Siliciumkarbid bzw. einer Probe aus Silikonmaterial beziehen. Die Verfahrensabläufe sind natürlich direkt in die entsprechende Behandlung von Stent-Trägern umzusetzen.

## Patentansprüche

1. Urologisches Implantat, insbesondere Gefäßwandstütze für den Urinal-trakt, mit einem vorzugsweise im wesentlichen röhrenförmigen, eigenstabilen Träger (8),
**gekennzeichnet durch**
eine an der Trägeroberfläche gebundene Spacer-Lage (6) und eine an der Spacer-Lage (6) gebundene Inhibitor-Lage (15) aus einem Glykosaminoglycan.

2. Implantat nach Anspruch 1, **dadurch gekennzeichnet, daß** der Träger aus einem Polymer, insbesondere Silikon, Polyurethan oder Polyvinylchlorid besteht.

3. Implantat nach Anspruch 1, **dadurch gekennzeichnet, daß** der Träger aus einem eigenstabilen Gerüst, insbesondere aus Metall mit einer Beschichtung aus einem Polymer, insbesondere Silikon, Polyurethan oder Polyvinylchlorid besteht.

4. Implantat nach Anspruch 1, **dadurch gekennzeichnet, daß** der Träger (8) aus Tantal, einer Titanlegierung, medizinischem Stahl oder pyrolytischem Kohlenstoff jeweils mit einer amorphen Siliciumkarbid-Beschichtung (13) als aktivierter Substrat-Oberfläche besteht.

5. Implantat nach Anspruch 2 oder 3, **dadurch gekennzeichnet, daß** die Spacer-Lage auf der Basis einer Propylsiloxyl-Verbindung gebildet ist.

6. Implantat nach Anspruch 5, **dadurch gekennzeichnet, daß** die Spacer-Lage aus einer partiell substituierten 3-(Adipinsäureamido)Propylsiloxyl-Verbindung gebildet ist.

7. Implantat nach Anspruch 4, **dadurch gekennzeichnet, daß** die Spacer-Lage (6) auf der Basis einer photoaktiven Benzophenon-Verbindung gebildet ist.

8. Implantat nach Anspruch 7, **gekennzeichnet durch** eine Fmoc-p-Bz-Phe-OH-Lösung in N, N'-Dimethylformamid als photoaktive Benzophenonverbindung.

9. Implantat nach einem der Ansprüche 1 bis 8, **dadurch gekennzeichnet, daß** die Inhibitor-Lage (15) aus einer auf der Spacer-Lage (6) immobilisierten Heparin-Lage (15) gebildet ist.

10. Implantat nach Anspruch 7 oder 8, **dadurch gekennzeichnet, daß** eine Heparin-Lage als Inhibitor-Lage (15) mittels einer kovalenten Peptid-Bindung auf der Substratoberfläche angelagert und immobilisiert ist.

## Claims

1. A urological implant, in particular a vascular wall support for the urinary tract, comprising a preferably substantially tubular, inherently stable carrier (8),
**characterized by**
a spacer film (6) bonded to the carrier surface and an inhibitor film (15) of a glycosamino glycan bonded to the spacer film (6).

2. An implant according to claim 1, **characterized in that** the carrier (8) consists of a polymer, in particular silicone, polyurethane or polyvinyl chloride.

3. An implant according to claim 1, **characterized in that** the carrier consists of an inherently stable framework, in particular of metal with a coating of a polymer, in particular silicone, polyurethane or polyvinyl.

4. An implant according to claim 1, **characterized in that** the carrier (8) consists of tantalum, a titanium alloy, medical steel or pyrolytic carbon, each of which having an amorphous silicon-carbide coating (13) as an activated substrate surface.

5. An implant according to claim 2 or 3, **characterized in that** the spacer film is formed on the basis of a propyl-siloxyl compound.

6. An implant according to claim 5, **characterized in that** the spacer film is formed from a partially substituted 3-(adipinic-acid-amido)-propylsiloxyl compound.

7. An implant according to claim 4, **characterized in that** the spacer film (6) is formed on the basis of a photoactive benzophenone compound.

8. An implant according to claim 7, **characterized by** an Fmoc-p-Bz-Phe-OH solution in N,N'-dimethylformamide as a photoactive benzophenone compound.

9. An implant according to one of claims 1 to 8, **characterized in that** the inhibitor film (15) is formed from a heparin layer (15) immobilized on the spacer film (6).

10. An implant according to claim 7 or 8, **characterized in that** a heparin layer is deposited and immobilized as an inhibitor film (15) on the substrate surface by means of a covalent peptide bond.

## Revendications

1. Implant urologique, en particulier une armature de paroi vasculaire pour le tract urinaire avec un support (8) autostable, de préférence, sensiblement de forme tubulaire, **caractérisé par** une couche intercalaire (6) liée à la surface du support et une couche inhibitrice (15) constituée de glycosaminoglycane, liée à la couche intercalaire (6).

2. Implant selon la revendication 1, **caractérisé en ce que** le support est constitué d'un polymère, en particulier de silicone, de polyuréthanne ou de poly-(chlorure de vinyle).

3. Implant selon la revendication 1, **caractérisé en ce que** le support est constitué d'une structure autostable en particulier constituée de métal avec un revêtement d'un polymère, en particulier de silicone, de polyuréthanne ou de poly(chlorure de vinyle).

4. Implant selon la revendication 1, **caractérisé en ce que** le support (8) est constitué de tantale, d'un alliage de titane, d'acier médicinal ou de carbone pyrolytique, à chaque fois avec un revêtement (13) de carbure de silicium amorphe en tant que surface de substrat activée.

5. Implant selon la revendication 2 ou 3, **caractérisé en ce que** la couche intercalaire est formée sur la base d'un composé propylsiloxyle.

6. Implant selon la revendication 5, **caractérisé en ce que** la couche intercalaire est formée d'un composé 3-(adipoylamido)propylsiloxyle.

7. Implant selon la revendication 4, **caractérisé en ce que** la couche intercalaire (6) est formée sur la base d'un composé benzophénone.

8. Implant selon la revendication 7, **caractérisé par** une solution de Fmoc-p-Bz-Phe-OH dans du N,N'-diméthylformamide en tant que composé benzophénone photoactif.

9. Implant selon l'une quelconque des revendications 1 à 8, **caractérisé en ce que** la couche inhibitrice (15) est formée d'une couche d'héparine (15) immobilisée sur la couche intercalaire (6)

10. Implant selon la revendication 7 ou 8, **caractérisé en ce qu'**une couche d'héparine (15) en tant que couche inhibitrice est fixée par addition et immobilisée sur la surface du substrat au moyen d'un composé peptidique.
